(19) Europäisches Patentamt / European Patent Office / Office européen des brevets

(11) **EP 3 655 801 B1**

(12) **FASCICULE DE BREVET EUROPEEN**

(45) Date de publication et mention de la délivrance du brevet:
**12.10.2022 Bulletin 2022/41**

(21) Numéro de dépôt: **18755520.6**

(22) Date de dépôt: **17.07.2018**

(51) Classification Internationale des Brevets (IPC):
**G01V 3/165** (2006.01)      **A61B 5/06** (2006.01)

(52) Classification Coopérative des Brevets (CPC):
**G01V 3/165; A61B 5/062**

(86) Numéro de dépôt international:
**PCT/FR2018/051815**

(87) Numéro de publication internationale:
**WO 2019/016465 (24.01.2019 Gazette 2019/04)**

(54) **PROCÉDÉ DE LOCALISATION D'UN OBJET ÉVOLUANT DANS UN CHAMP MAGNÉTIQUE GÉNÉRÉ PAR UN ENSEMBLE D'AU MOINS TROIS GÉNÉRATEURS DE CHAMP MAGNÉTIQUES**

VERFAHREN ZUR ORTUNG EINES OBJEKTES WELCHES SICH IN EINEM MAGNETFELD BEWEGT DAS DURCH EINE ANORDNUNG VON MINDESTENS DREI MAGNETFELDGENERATOREN ERZEUGT WIRD

METHOD FOR LOCATING AN OBJECT MOVING IN A MAGNETIC FIELD GENERATED BY AN ASSEMBLY OF AT LEAST THREE MAGNETIC FIELD GENERATORS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.07.2017 FR 1756755**

(43) Date de publication de la demande:
**27.05.2020 Bulletin 2020/22**

(73) Titulaire: **SYSNAV**
**27200 Vernon (FR)**

(72) Inventeurs:
• **VISSIERE, David**
**75009 Paris (FR)**
• **HILLION, Mathieu**
**27200 Vernon (FR)**
• **MEIER, Hendrik**
**27200 Vernon (FR)**

(74) Mandataire: **Regimbeau**
**20, rue de Chazelles**
**75847 Paris Cedex 17 (FR)**

(56) Documents cités:
CN-A- 104 776 865      US-A1- 2011 224 537
US-A1- 2015 057 969      US-B1- 8 311 767

**Description**

DOMAINE TECHNIQUE GENERAL

La présente invention concerne le domaine de la navigation sans GPS.

Plus précisément, elle concerne un procédé de localisation d'un objet évoluant dans un champ magnétique artificiel.

ETAT DE L'ART

On appelle la localisation magnétique une technique dans laquelle un objet équipé d'au moins un capteur de champ magnétique est localisé dans un champ magnétique artificiel, généré par au moins une source. La localisation magnétique permet une haute précision (avec une erreur bien sous-millimétrique et sous un degré). Des applications se trouvent principalement dans le médical, par exemple pour de la navigation chirurgicale.

La source, i.e. un générateur de champ magnétique, contient typiquement trois bobines perpendiculaires qui émettent des champs magnétiques distinguables, par exemple des champs AC avec des fréquences différentes. Le capteur mesure les champs magnétiques selon trois axes également. Les signaux du capteur selon ses axes sont par la suite démodulés par rapport aux fréquences des bobines émettrices. L'ensemble des données résultant de ce processus et la maîtrise (modélisation et calibration) du champ magnétique permettent de calculer la position et l'orientation du capteur (ou d'un outil sur lequel le capteur est fixé) en temps réel.

La technique pour estimer la position et l'orientation d'un objet en mesurant le champ magnétique émis par une source avec un capteur magnétique est bien connue depuis les années 1970, voir par exemple les brevets US3868565, US4737794, ou US4945305.

Le traitement du signal reçu par le capteur implique sa décomposition en des composantes issues de chaque des bobines de la source. Cette décomposition peut être réalisé en utilisant : (1) des champs magnétiques DC pulsés de manière que les trois bobines de la source émettent un signal tour à tour (voir par exemple le brevet US4945305) ; (2) des champs magnétiques AC avec des fréquences différentes pour les trois bobines de la source, permettant de les distinguer par une démodulation (voir par exemple le brevet US3868565). La décomposition des signaux reçus par les bobines triaxiales du capteur par rapport aux bobines triaxiales émettrices fournit une matrice de $3{\times}3=9$ coefficients à base de laquelle la position et l'attitude (soit six degrés de liberté au total) du capteur relativement à la source peuvent être calculées (voir US4737794).

Il y a principalement deux difficultés rencontrées dans de tels systèmes source - capteur.

Tout d'abord, comme le champ magnétique décroît avec le cube de la distance source - capteur, le signal devient rapidement faible et atteint typiquement le niveau du bruit à une distance d'environ un mètre pour les systèmes sur le marché. On a ainsi une dégradation de la performance en fonction de la distance. Une bonne précision ne peut être atteinte que dans une région limitée autour de la source. Une solution est d'agrandir la taille des bobines ou augmenter le courant, ce qui est en pratique rapidement limité.

Ensuite, un champ magnétique émis par la source, qu'il soit DC pulsé ou AC, est perturbé par (1) des matériaux conducteurs (par l'induction des courants de Foucault) et (2) des matériaux perméables, les deux matériaux pouvant être rencontrés dans les applications. De telles perturbations faussent les mesures et dégradent la performance.

Pour les perturbations immobiles, une solution possible est de dresser une carte du champ magnétique selon un réseau 3D dans l'espace. Cette solution demande d'effectuer une calibration par un expert et n'est pas pratique du tout dans toutes les situations où les objets perturbants comme des outils sont régulièrement déplacés, ce qui demanderait une recalibration en permanence.

Alternativement, il est proposé dans le brevet US6636757 de monter jusqu'à trois sources triaxe sur un « bouclier » qui agit comme un élément isolant limitant la distorsion du champ magnétique due à un objet métallique perturbateur. Un procédé de calibration associé est prévu. On comprend qu'une telle solution est probablement efficace mais très lourde en pratique.

Dans le brevet US6400139, il est proposé l'utilisation d'une seule source et de deux capteurs : un capteur « témoin » et celui dont la position est cherchée (« probe »), considéré comme une source secondaire de champ AC. En d'autres termes, l'architecture proposée est équivalente à une architecture avec deux sources et un capteur mais dans laquelle on chercherait la position d'une source, celle du capteur étant connue. Un examen de la phase du signal capteur permet de séparer les perturbations dues aux matériaux conducteurs, mais pas celles des matériaux perméables.

Dans d'autres documents, il n'est pas tenté de corriger l'effet d'une perturbation, mais simplement de la détecter, voir les demandes US2011224537 ou US2011004430.

On constate que la recherche d'une solution qui soit à la fois applicable et sans perte de performance ou de résolution temporelle dans une situation générique reste un problème ouvert.

Il serait ainsi souhaitable de disposer d'une méthode de localisation magnétique qui soit toujours aussi efficace,

mais de surcroît complètement robuste contre les perturbations de quelque nature que ce soit.

PRESENTATION DE L'INVENTION

La présente invention se rapporte ainsi selon un premier aspect à un procédé de localisation d'un objet solidaire d'un capteur magnétique triaxe, évoluant dans un champ magnétique généré par un ensemble d'au moins trois générateurs magnétiques triaxe statiques dans un référentiel commun, le procédé étant caractérisé en ce qu'il comprend des étapes de :

(a) Pour chaque générateur détermination en fonction de mesures de champ magnétique acquises par le capteur et associées audit générateur d'une position dudit capteur dans ledit référentiel commun ;
(b) Calcul pour chaque générateur d'un paramètre représentatif d'une erreur sur ladite position déterminée du capteur pour le générateur ;
(c) Sélection d'un sous-ensemble de l'ensemble des générateurs en fonction desdits paramètres estimés pour chacun des générateurs ;
(d) Estimation de la position de l'objet par fusion des positions déterminées du capteur pour chaque générateur sélectionné dans ledit sous-ensemble.

Selon d'autres caractéristiques avantageuses et non limitatives :

- le capteur et les générateurs sont chacun constitué de trois bobines organisées en triaxes ;
- l'étape (b) comprend pour chaque générateur l'estimation des valeurs de deux invariants mathématiques en fonction des mesures de champ magnétique acquises par le capteur et associées audit générateur ;
- ledit paramètre représentatif d'une erreur sur ladite position déterminée du capteur est calculé pour chaque générateur à l'étape (b) en fonction des valeurs estimées desdits deux invariants mathématiques pour ledit générateur et de valeurs théoriques desdits deux invariants mathématiques ;
- ledit paramètre représentatif d'une erreur sur ladite position déterminée du capteur est une estimation de ladite erreur sur ladite position déterminée du capteur ;
- ledit sous-ensemble de l'ensemble des générateurs sélectionné à l'étape (c) comprend chaque générateur pour lequel ledit paramètre estimé est inférieur à au moins un seuil de référence ;
- si ledit sous-ensemble comprend moins qu'un nombre minimal prédéterminé de générateurs, ledit sous-ensemble de l'ensemble des générateurs sélectionné à l'étape (c) comprend en outre chaque générateur pour lequel ledit paramètre estimé est inférieur à au moins un seuil dégradé supérieur au seuil de référence ;
- la position obtenue par fusion est la position moyenne des positions déterminées du capteur pour chaque générateur sélectionné dans ledit sous-ensemble ;
- si ledit sous-ensemble de l'ensemble des générateurs sélectionné à l'étape (c) comprend au moins un générateur pour lequel ledit paramètre estimé est supérieur audit seuil de référence, la position obtenue par fusion est la position moyenne des positions déterminées du capteur pour chaque générateur du plus grand sous-ensemble dudit sous-ensemble des générateurs pour lesquels ledit paramètre estimé reste inférieur audit seuil de référence ;
- l'attitude est prise en compte en surplus de la position dans les étapes (a) à (d).

Selon un deuxième aspect, l'invention concerne un système comprenant un capteur magnétique triaxe, un ensemble d'au moins trois générateurs magnétiques triaxe statiques dans un référentiel commun, pour la localisation d'un objet solidaire du capteur et évoluant dans le champ magnétique généré par lesdits générateurs, caractérisé en ce qu'il comprend des moyens de traitement de données configurés pour mettre en œuvre :

- Un module de détermination, pour chaque générateur, en fonction de mesures de champ magnétique acquises par le capteur et associées audit générateur, d'une position dudit capteur dans ledit référentiel commun ;
- Un module de calcul pour chaque générateur d'un paramètre représentatif d'une erreur sur ladite position déterminée du capteur pour le générateur ;
- Un module de sélection d'un sous-ensemble de l'ensemble des générateurs en fonction desdits paramètres estimés pour chacun des générateurs ;
- Un module d'estimation de la position de l'objet par fusion des positions déterminées du capteur pour chaque générateur sélectionné dans ledit sous-ensemble.

Selon un troisième et un quatrième aspect, l'invention concerne un produit programme d'ordinateur comprenant des instructions de code pour l'exécution d'un procédé de localisation d'un objet solidaire d'un capteur magnétique triaxe, évoluant dans un champ magnétique généré par un ensemble d'au moins trois générateurs magnétiques triaxe statiques dans un référentiel commun, selon le premier aspect de l'invention ; et un moyen de stockage lisible par un équipement informatique sur lequel un produit programme d'ordinateur comprend des instructions de code pour l'exécution d'un procédé de localisation d'un objet solidaire d'un capteur magnétique triaxe, évoluant dans un champ magnétique généré par un ensemble d'au moins trois générateurs magnétiques triaxe statiques dans un référentiel commun, selon le premier aspect de l'invention.

## PRESENTATION DES FIGURES

D'autres caractéristiques et avantages de la présente invention apparaîtront à la lecture de la description qui va suivre d'un mode de réalisation préférentiel. Cette description sera donnée en référence aux dessins annexés dans lesquels :

- la figure 1 est un schéma d'un système pour la mise en œuvre du procédé selon l'invention ;
- La figure 2 illustre plus en détail un exemple de mise en œuvre du procédé selon l'invention.

## DESCRIPTION DETAILLEE

*Architecture*

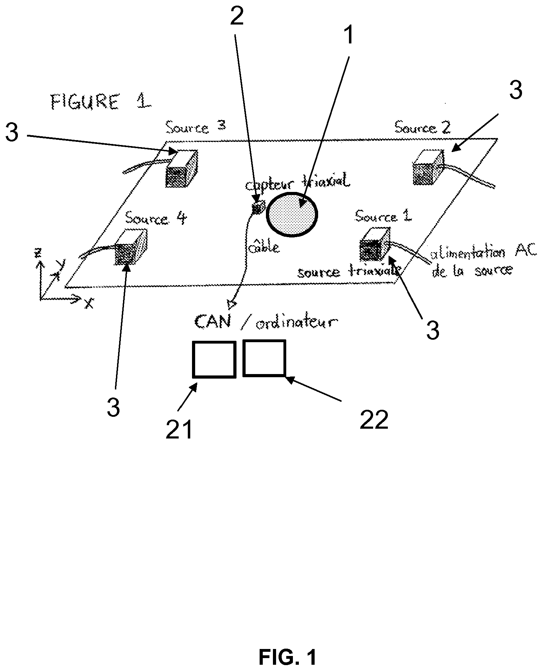

En référence à la **figure 1**, le présent procédé permet la localisation d'un objet 1 solidaire d'un capteur magnétique triaxe 2, évoluant dans un champ magnétique (noté $\vec{B}$) généré par un ensemble d'au moins trois générateurs magnétiques triaxe 3 (dans la suite de la présente description, on appellera n le nombre de générateurs 3, avec $n \leq 3$, dans l'exemple de la figure 1 $n = 4$. On pourra aller jusqu'à huit ou dix générateurs 3 pour des résultats d'une très bonne qualité sans qu'il soit trop difficile de distinguer les contributions des différents générateurs, voir plus loin).

Lesdits générateurs magnétiques triaxes 3 sont statiques dans un référentiel commun, préférentiellement orthonormé, typiquement le référentiel terrestre. Cela signifie qu'ils sont fixes et ont donc des positions prédéterminées les uns parmi les autres. Les triaxes sont avantageusement tous orientés conformément audit repère commun orthonormé, i.e. que les trois axes de chaque générateur 3 correspondent aux trois axes dudit repère commun, de sorte à faciliter les calculs.

Ils sont typiquement constitués de trois bobines axiales organisées en triaxe (i.e. s'étendant chacune selon un des trois axes). De façon préférée, les générateurs (et même chacune de leurs bobines) sont associés à des fréquences différentes et distinguables (fréquence du courant alternatif les alimentant), mais on comprendra comme expliqué dans l'introduction qu'on peut aussi par exemple pulser les champs dans les différents générateurs tour à tour.

On comprendra que ce champ magnétique « artificiel » généré par les générateurs 3 s'inscrit généralement dans un champ magnétique ambiant (d'origine naturelle), sensiblement statique dans ledit référentiel commun, qui n'est pas généré par les générateurs 3 (typiquement le champ magnétique terrestre), mais on comprendra que ce champ ambiant est négligeable devant le champ généré par les générateurs, de sorte que ce dernier pourra être considéré seul.

Comme déjà expliqué, le champ magnétique est un champ vectoriel en l'espèce tridimensionnel, c'est-à-dire associant un vecteur de dimension trois à chaque point de tridimensionnel dans lequel l'objet est mobile.

Cet objet 1 peut être n'importe quel objet mobile dont la connaissance de la position est souhaitée, par exemple un véhicule à roues, un drone, un outil, un instrument, etc., mais également une personne ou une partie de son corps (ses mains, etc.).

L'objet 1 est équipé d'un capteur magnétique triaxe 2, i.e. de moyens de mesure magnétiques, généralement constitué de trois bobines (et de façon générale des magnétomètres) axiales disposées en triaxe. Plus précisément, le capteur 2 est solidaire de l'objet 1, i.e. il présente un mouvement sensiblement identique dans le référentiel terrestre. De façon préférée, le référentiel de l'objet 1 est muni d'un repère cartésien orthonormé dans lequel les coordonnées sont notées $(x_1, x_2, x_3)$, le capteur 20 présente ainsi une position prédéterminée et fixe dans ce repère.

De façon préférée, le repère orthonormé associé à l'objet 1 est choisi par convention (et par facilité pour la suite de la présente description) tel que le triaxe du capteur 2 est avantageusement orienté conformément audit repère orthonormé associé à l'objet 1, i.e. que les trois axes du capteur 2 correspondent aux trois axes dudit repère de l'objet 1, de sorte à faciliter les calculs.

Mais l'homme du métier saura dans tous les cas transposer à n'importe quelle disposition spatiale des générateurs 3 et capteurs 2.

Le système peut comprendre des moyens de traitement 21 (typiquement un processeur) pour la mise en œuvre directement en temps réel des traitements du présent procédé, ou bien les mesures peuvent être émises via des moyens de communication vers un dispositif externe tel qu'un serveur distant, ou encore les mesures peuvent être enregistrées dans des moyens de stockage de données 22 locaux (une mémoire par exemple de type flash) pour un éventuel traitement a posteriori.

Dans l'exemple de la figure 1, le capteur 2 et les générateurs 3 sont disposés sur une table, laquelle est équipée des moyens de traitement 21, avec lesquels ils sont connectés via une connexion filaire.

Si c'est un équipement distant (tel qu'un serveur) qui héberge « l'intelligence », il comprend des moyens de traitement dédiés tels qu'un processeur pour la mise en œuvre des traitements du présent procédé qui vont être décrits.

Dans la suite de la présente description, on verra que les moyens de traitement de données locaux ou d'un équipement distant peuvent indifféremment et selon les applications réaliser tout ou partie des étapes du procédé.

*Détermination de position mono-source*

Dans une première étape (a), le procédé comprend la détermination, pour chaque générateur 3, d'une position dudit capteur 2 dans ledit référentiel commun en fonction de mesures de champ magnétique acquises par le capteur 2 et associées audit générateur 3. L'étape (a) comprend avantageusement également la détermination, pour chaque générateur 3, d'une attitude (i.e. une orientation) dudit capteur 2 dans ledit référentiel commun.

En d'autres termes, $n$ positions/attitudes sont indépendamment déterminées. Chacune de ces déterminations de position/attitude est avantageusement conforme à l'état de l'art « mono-source » qui comporte un générateur consistant en trois bobines orthogonales et un capteur également de trois bobines triaxiales. En d'autres termes, chaque position/attitude est calculée comme si le générateur 3 associé était tout seul.

Chaque « sous-système » générateur - capteur du présent système multi-source est ainsi typiquement équivalent à un système mono-source tel que décrit dans le brevet US4737794. Il permet une localisation dans une région limitée autour de la source, par exemple avec les méthodes rapportées dans les brevets US4737794 et US5307072.

On rappelle que les contributions des divers générateurs 3 sont préférentiellement différentiées grâce à leurs fréquences différentes.

Sont préférentiellement extraits les six degrés de liberté pour la localisation en position et en attitude de l'objet 1 des neuf coefficients d'une matrice (appelée « signal matrix » dans les documents susmentionnée) issue de la démodulation des signaux reçus par les trois bobines du capteur 2 par rapport aux trois bobines du générateur 3.

Comme l'on va voir, le présent système multi-source va « fusionner » les résultats de localisation par chaque sous-système mono-source d'une manière astucieuse pour obtenir une position/orientation « unifiée ».

Il est à noter que le procédé comprend préférentiellement l'harmonisation préalable des générateurs 3 pour pouvoir représenter les localisations par rapport à toutes les sources dans le référentiel commun. En effet le référentiel commun est indispensable pour vérifier la compatibilité mutuelle des résultats mono-source et les fusionner de manière intelligente.

L'harmonisation est réalisée en calibration. On détermine les positions et les attitudes de toutes les sources par rapport à un repère absolu, qui peut être choisi selon l'application.

Dans la suite de la description, on ne parlera que de la position de l'objet 1, mais on comprendra que le présent procédé prend avantageusement également en considération l'attitude, et l'homme du métier saura transposer en conséquence.

*Intégrité mono-source*

Dans une étape (b), est estimée par les moyens de traitement de données 21 pour chaque générateur 3 un paramètre représentatif d'une erreur sur ladite position (et le cas échant sur ladite attitude) déterminée du capteur 2 pour le générateur 3. En d'autres termes, est associée à chacun des $n$ générateurs 3 un paramètre d'erreur. Ledit paramètre peut être vu comme un niveau d'erreur, et peut être directement une estimation de l'erreur de position (exprimée comme une distance) et/ou de l'erreur d'attitude (exprimée comme un angle).

Ce paramètre permet de distinguer les sources dont l'intégrité est compromise (erreur anormalement haute, par exemple du fait de la présence d'une perturbation métallique à proximité du générateur 3), de celles dont l'intégrité est confirmée (erreur dans des niveaux de tolérance acceptables).

En effet, la demanderesse a constaté le fait physique selon lequel dans un système mono-source, l'erreur due à une perturbation locale est asymétrique par rapport au centre de la perturbation : Elle est plus grande sur le côté éloigné de la source que sur le côté entre la source et la perturbation. En d'autres termes, une perturbation locale disposée entre la source et le capteur va avoir un fort impact sur la mesure, mais une perturbation locale disposée au-delà du capteur de va pas avoir d'impact sensible sur la mesure associé à cette source.

Ainsi, le présent procédé propose non pas de chercher à corriger les perturbations ni même à les détecter comme c'était le cas dans l'art antérieur, mais simplement de jouer sur la redondance des générateurs 3 de sorte à baser en permanence la localisation uniquement sur la base des mesures fiables, i.e. d'exclure les « sources perturbées ».

Dès lors que l'on a un arrangement des générateurs adapté aux endroits typiques des perturbations (par exemple en entourant la zone dans laquelle l'objet 1 est destiné à se déplacer), une telle solution est très robuste (tout type de perturbation, y compris des éventuelles déficiences d'un générateur 3) et très facile à mettre en œuvre. De plus et comme l'on va voir plus loin, il est modulable en fonction des niveaux de précision souhaités (selon l'application recherchée).

En expérience, une réduction de l'erreur par un facteur entre deux et cinq est observée.

Pour cela, comme décrit en haut, le calcul mono-source extrait les six degrés de liberté pour la localisation en position et en attitude des neuf coefficients d'une matrice issue de la démodulation des signaux reçus par les trois bobines du capteur par rapport aux trois bobines du générateur 3. Or, parmi les degrés de liberté redondants (trois), il y a deux invariants mathématiques, c'est-à-dire des grandeurs qui ont théoriquement des valeurs constantes. L'estimation de ces invariants avec les données mesurées permet de tester l'intégrité de la mesure (en comparant aux valeurs théoriques qu'ils auraient dues présenter dans un modèle parfait) et ainsi de fournir une estimation de l'erreur.

Nous appelons $M_{ij}$ l'amplitude du champ magnétique issu de la bobine $j$ d'un générateur 3 et mesuré par la bobine $i$ du capteur 2, avec $i,j$ = 1,2,3 pour les axes $x$, $y$, $z$ des deux repères. On note qu'on a $n$ matrices $M_k$, chacune associée à un des $n$ générateurs 3.

Si on suppose que chaque générateur 3 comporte trois bobines parfaitement orthogonales qui émettent un champ magnétique parfaitement dipolaire et que le capteur triaxial est également parfait, chaque matrice M a la forme (voir par exemple US4737794) :

$$M = \mathbf{A}^{\mathrm{T}} \cdot \frac{k}{r^5} \begin{pmatrix} 3x^2 - r^2 & 3xy & 3xz \\ 3xy & 3y^2 - r^2 & 3yz \\ 3xz & 3yz & 3z^2 - r^2 \end{pmatrix}.$$

Ici, A dénote la matrice orthogonale pour l'attitude du capteur par rapport au repère de la source. Le paramètre k est le moment dipolaire multiplié avec $\mu/4\pi$ et $r = \sqrt{x^2 + y^2 + z^2}$ et la distance entre la source et le capteur, le dernier se trouvant en $(x,y,z)$ dans le repère de la source.

Une décomposition en valeur singulière de la matrice M donne

$$M = P \begin{pmatrix} s_1 & 0 & 0 \\ 0 & s_2 & 0 \\ 0 & 0 & s_3 \end{pmatrix} Q^{\mathrm{T}},$$

où $P,Q$ sont des matrices orthogonales alors que les valeurs singulières valent $s_1 = 2k/r^3$ et $s_2 = s_3 = k/r^3$. Les ratios $s_1/s_2$ = 2 et $s_1/s_3$ = 2 ont donc des valeurs prédéfinies et sont lesdits invariants mathématiques inhérents au modèle dipolaire pour le champ magnétique. On comprendra que l'homme du métier pourra trouver d'autre valeurs d'invariants mathématiques, par exemple $s_2/s_1$ = 0,5 et $s_2/s_3$ = 1, $s_1$ - $2s_2$ = 0 et $s_1$ - $2s_3$ = 0, etc. De façon générale, on pourra prendre tout invariant mathématique obtenu à partir des valeurs singulières de la matrice des données mesurées de champ magnétique.

Une déviation des invariants par rapport à leurs valeurs théorique indique que le modèle n'est pas valable, ce qui peut être dû à une perturbation du champ magnétique (ou tout autre problème lié au générateur 3).

Pour quantifier la déviation, on peut par exemple, si on note $c_1^{est}, c_1^{th}, c_2^{est}, c_2^{th}$ les valeurs estimées et théoriques du premier et du deuxième invariants, introduire la fonction :

$$f(M) = \left(c_1^{th} - c_1^{est}\right)^2 + \left(c_2^{th} - c_2^{est}\right)^2 = \left(2 - \frac{s_1}{s_2}\right)^2 + \left(2 - \frac{s_1}{s_3}\right)^2$$

dans notre exemple préféré ; ou des fonctions similaires, qui permettent d'estimer les déviations des invariants mathématiques $s_1/s_2$ et $s_1/s_3$. Plus précisément, on pourra choisir toute fonction telle que dans le cas parfait (i.e. $\frac{s_1}{s_2} = \frac{s_1}{s_3} = 2$) $f(M)$ = 0, et sinon $f(M)$ > 0, avec $f$ croissante lorsque l'écart entre les valeurs estimées des invariants (à partir des données mesurées) et leurs valeurs théoriques (en l'espèce 2) augmente.

Par exemple, on pourra prendre alternativement la fonction $f(M) = \left| c_1^{th} - c_1^{est} \right| + \left| c_2^{th} - c_2^{est} \right|$.

La fonction *f(M)* ou toute fonction similaire est un exemple préféré de paramètre représentatif d'une erreur de position/attitude.

La fonction *f(M)* permet même une estimation de l'erreur $\Delta x$ en position et l'erreur $\Delta \varphi$ en attitude :

$$\Delta x \approx C_1 \, r \, f(M) \, ;$$

$$\Delta \varphi \approx C_2 \, f(M) \, .$$

Les coefficients $C_1$ et $C_2$ peuvent être déterminés en simulation ou en expérience et adaptés au niveau de confiance souhaité.

Ainsi l'étape (b) comprend avantageusement le calcul de $\{f(M_k)\}_{k\in[\![1;n]\!]}$, voire celui de $\{\Delta x_k\}_{k\in[\![1;n]\!]}$ et/ou $\{\Delta \varphi_k\}_{k\in[\![1;n]\!]}$.

Dans une situation réaliste, les champs magnétiques ne correspondent pas aux dipôles parfaits. Par contre, les déviations du champ dipolaire peuvent être modélisées et corrigées en calibration et le critère d'intégrité sera calculé pour une matrice M corrigée. Dans ce cas, la fonction *f(M)* quantifie également la déviation du modèle et donc permet une estimation de l'erreur.

On notera que pour des raisons physiques, les méthodes mono-source ne permettent une localisation qu'avec une ambigüité sur le demi-espace, car le champ magnétique ne permet pas de distinguer les positions (*x,y,z*) et (*-x,-y,-z*) (dans le repère commun), cette contrainte est physiquement inévitable pour les champs dipolaires.

Le présent système multi-source ne souffre pas de cette ambigüité, car le mauvais demi-espace pour une ou plusieurs générateurs 3 mènerait à une incompatibilité des localisations mono-source avec des écarts de l'ordre du mètre alors que pour les bons demi-espaces, la compatibilité est typiquement vérifiée à l'ordre sous-millimétrique ou, au cas de perturbations (locales) à un ordre au moins centimétrique.

*Sélection et fusion multi-source*

Nous utilisons l'estimation mono-source de l'erreur pour présélectionner ces sources qui seront prises en compte dans le calcul multi-source.

Dans une étape (c) est sélectionné un sous-ensemble de l'ensemble des générateurs 3 en fonction desdits paramètres estimés pour chacun des générateurs 3. L'idée est d'écarter le cas échéant les éventuelles sources dont l'intégrité est compromise.

Une fois que les résultats pour la localisation du capteur par rapport à tous les générateurs 3 sélectionnés sont disponibles dans un repère commun, ils seront fusionnés dans un résultat final qui peut inclure une estimation de l'erreur. L'étape (d) comprend ainsi l'estimation de la position de l'objet 1 par fusion des positions déterminées du capteur 2 pour chaque générateur 3 sélectionné dans ledit sous-ensemble. Plus précisément, une position unifiée du capteur 2 est déterminée, et la position de l'objet 1 en est déduite (par un simple offset). Il faut bien comprendre que par fusion, on entend la combinaison de plusieurs mesures de position « complètes » calculées par rapport à des générateurs 3 en particulier triaxes. Cette disposition ne doit pas être confondue avec la combinaison de mesures magnétiques par rapport à plusieurs générateurs magnétiques monoaxes pour calculer une seule position comme l'on trouve dans l'état de l'art (cela ne constitue pas une fusion de plusieurs positions, mais la simple reconstitution d'une unique position).

Ledit sous-ensemble de l'ensemble des générateurs 3 sélectionné à l'étape (c) comprend préférentiellement chaque générateur 3 pour lequel ledit paramètre estimé est inférieur à au moins un seuil de référence, dit « cible de précision », par exemple 1 mm en position et/ou 1° en attitude.

Il peut être souhaitable que ledit sous-ensemble contienne au moins un nombre minimal prédéterminé *N* de générateurs 3, par exemple *N* = 2 (en particulier si *n* = 3) ou *N* = 3 (en particulier si *n* > 3).

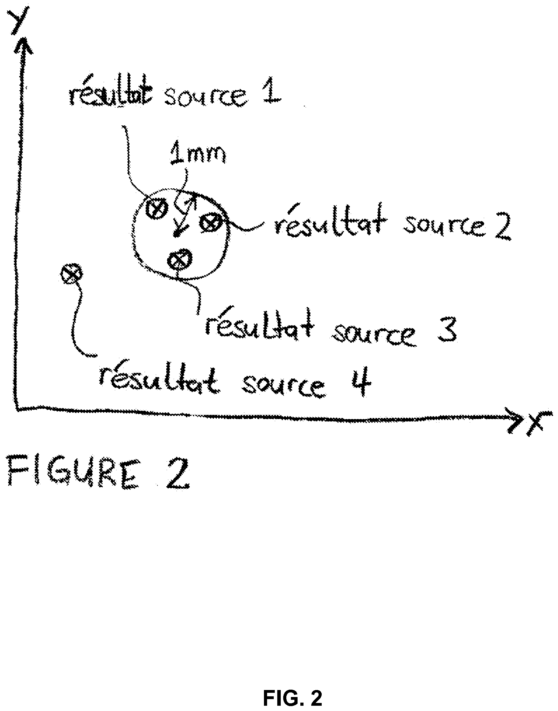

Si c'est le cas dudit sous-ensemble sélectionné, alors tout va bien et l'on peut passer à l'étape (d). Dans l'exemple de la **figure 2,** Les résultats pour la position des sources 1 - 3 sont compatibles par rapport à un seuil de référence de 1 mm. La source 4 n'est pas compatible, possiblement à cause d'une perturbation de son champ par un objet métallique entre le capteur et cette source. Cette perturbation, qui infligerait une erreur de quelques millimètres sur une localisation avec seulement la source 4, n'a pas d'impact sur la mesure multi-source.

Si ce n'est pas le cas (ledit sous-ensemble contient moins que le nombre minimal prédéterminé *N* de générateurs 3), il est souhaitable d'en rajouter même si d'autres générateurs 3 sont associés à une précision moindre.

Ainsi, si ledit sous-ensemble comprend moins qu'un nombre minimal prédéterminé de générateurs 3, ledit sous-ensemble de l'ensemble des générateurs 3 sélectionné à l'étape (c) comprend en outre au moins un (voire chaque) générateur 3 pour lequel ledit paramètre estimé est inférieur à au moins un seuil dégradé supérieur au seuil de référence, dite « cible dégradée » par exemple 1,5 mm en position et/ou 1,5° en attitude.

Il peut y avoir plusieurs seuils dégradés, et l'on peut considérer successivement ces seuils (du plus faible au plus élevé) jusqu'à ce que l'on atteigne ledit nombre minimal prédéterminé $N$ de générateurs.

En d'autres termes, ledit sous-ensemble de l'ensemble des générateurs 3 sélectionné à l'étape (c) comprend chaque générateur 3 pour lequel ledit paramètre estimé est inférieur au seuil le plus faible parmi une pluralité de seuils prédéterminés tel que ledit sous-ensemble comprend au moins un nombre minimal prédéterminé de générateurs 3.

On comprendra que d'autres modes de calcul pourront être pris en compte par l'homme du métier, par exemple en triant les générateurs 3 selon la valeur de l'erreur de position associée, en en prenant par exemple les $N$ avec l'erreur la plus faible.

En ce qui concerne la fusion de l'étape (d), une façon simple de procéder est de prendre la position moyenne des positions déterminées (et le cas échéant de prendre l'attitude moyenne des attitudes déterminées) du capteur 2 pour chaque générateur 3 sélectionné dans ledit sous-ensemble.

Cela peut n'être valide que si le sous-ensemble ne comprend que des générateurs 3 pour lesquels l'erreur de position est en dessous du seuil de référence.

Si au contraire ledit sous-ensemble de l'ensemble des générateurs 3 sélectionné à l'étape (c) comprend au moins un générateur 3 pour lequel ledit paramètre estimé est supérieur audit seuil de référence (i.e. inférieur à un seuil dégradé), il peut être déterminé la valeur fusionnée sera la moyenne du plus grand sous-ensemble compatible (i.e. ou, en cas d'égalité entre deux ou plusieurs sous-ensembles, la moyenne du sous-ensemble avec une (co-)variance minimale.

On comprendra que d'autres modes de calcul pourront être pris en compte par l'homme du métier, par exemple en pondérant les positions déterminées dans le calcul de la position unifiée selon si le générateur 3 associé présente une erreur de position au-dessus ou en-dessous du seuil de référence. On peut par exemple même envisager de pondérer les positions déterminées avec l'inverse de l'erreur.

De façon particulièrement préféré, l'algorithme suit ainsi les points suivants pour chaque échantillon de mesure :

1. Choix d'un seuil de référence, par exemple 1 mm / 1°
2. Présélection des générateurs 3. Seuls ces générateurs pour lesquelles l'estimation de l'erreur est compatible avec le seuil de référence seront acceptés pour la suite. Si le nombre de générateurs 3 présélectionnées est inférieur au nombre minimal prédéterminé (par exemple trois), l'algorithme retourne au point 1 avec un seuil dégradé, par exemple 1.5 mm / 1.5°.
3. Test si les résultats des générateurs 3 présélectionnées sont compatibles par rapport au seuil prédéterminé.

   a. Si oui, le résultat sera la moyenne des positions associées aux générateurs 3 présélectionnés avec l'estimation de l'erreur donnée par le seuil de référence.
   b. Si non, tester si un sous-ensemble des générateurs 3 présélectionnés d'un nombre $N'$ égal ou inférieur au nombre prédéfini $N$ (par exemple $N' = 2$) est compatible par rapport au seuil de référence.

      i. Si oui, le résultat sera la moyenne des positions associées aux générateurs 3 du plus grand sous-ensemble compatible ou, en cas d'égalité entre deux ou plusieurs sous-ensembles, la moyenne des positions associées aux générateurs 3 du sous-ensemble avec une (co-)variance minimale. L'erreur estimée sera la cible de précision.
      ii. Si non, l'algorithme retournera au point 1 avec un seuil dégradé.

L'algorithme est modifiable par rapport au niveau de confiance (qui entre dans les tests de compatibilité), le nombre minimal prédéterminé N et les seuils.

Si l'algorithme ne trouve pas de localisation compatible avec le pire des seuils (dégradés), seul un seuil inférieur de l'erreur pourra être fourni. En adaptant les seuils à l'échelle des erreurs attendues ou tolérées, ce cas peut être évité ou ne présente pas de défaut. Si, par exemple, une précision meilleure que 1 mm est la seule contrainte pour le fonctionnement d'une application, il suffit de tester la cible de 1 mm et, si elle ne peut pas être atteinte à cause d'une perturbation, de rejeter la mesure.

*Equipements et système*

Selon un deuxième aspect, l'invention concerne en particulier un système pour la mise en œuvre de l'un ou l'autre des modes de réalisation du procédé.

Comme expliqué précédemment, le système comprend un capteur magnétique triaxe 2 et un ensemble d'au moins trois générateurs magnétiques triaxe 3 statiques dans un référentiel commun, pour la localisation d'un objet 1

solidaire du capteur 2 et évoluant dans le champ magnétique généré par lesdits générateurs 3.

Le système comprend, de façon éventuellement déportée, les moyens de traitement de données 21 configurés pour la mise en œuvre des étapes du procédé, et le cas échéant des moyens de stockage de données 22 et/ou des moyens de communication pour l'exportation des résultats.

Les moyens de traitement de données 21 du système sont configurés pour mettre en œuvre :

- Un module de détermination, pour chaque générateur 3, d'une position dudit capteur 2 dans ledit référentiel commun en fonction de mesures de champ magnétique acquises par le capteur 2 et associées audit générateur 3;
- Un module de calcul, pour chaque générateur 3, d'un paramètre représentatif d'une erreur sur ladite position déterminée du capteur 2 pour le générateur 3 ;
- Un module de sélection d'un sous-ensemble de l'ensemble des générateurs 3 en fonction desdits paramètres estimés pour chacun des générateurs 3 ;
- Un module d'estimation de la position de l'objet 1 par fusion des positions déterminées du capteur 2 pour chaque générateur 3 sélectionné dans ledit sous-ensemble.

*Produit programme d'ordinateur*

Selon un troisième et un quatrième aspects, l'invention concerne un produit programme d'ordinateur comprenant des instructions de code pour l'exécution (sur les moyens de traitement 21) d'un procédé de localisation d'un objet 1 solidaire d'un capteur magnétique triaxe 2, évoluant dans un champ magnétique généré par un ensemble d'au moins trois générateurs magnétiques triaxe 3 statiques dans un référentiel commun selon le premier aspect de l'invention, ainsi que des moyens de stockage lisibles par un équipement informatique (par exemple des moyens de stockage de données 22) sur lequel on trouve ce produit programme d'ordinateur.

**Revendications**

1. Procédé de localisation d'un objet (1) solidaire d'un capteur magnétique triaxe (2), évoluant dans un champ magnétique généré par un ensemble d'au moins trois générateurs magnétiques triaxe (3) statiques dans un référentiel commun, le procédé comprenant l'étape de :

   (a) Pour chaque générateur (3) détermination en fonction de mesures de champ magnétique acquises par le capteur (2) et associées audit générateur (3) d'une position dudit capteur (2) dans ledit référentiel commun ;

   le procédé étant **caractérisé en ce qu'**il comprend des étapes de :

   (b) Calcul pour chaque générateur (3) d'un paramètre représentatif d'une erreur sur ladite position déterminée du capteur (2) pour le générateur (3) ;
   (c) Sélection d'un sous-ensemble de l'ensemble des générateurs (3) en fonction desdits paramètres estimés pour chacun des générateurs (3), ledit sous-ensemble de l'ensemble des générateurs (3) sélectionné comprenant chaque générateur (3) pour lequel ledit paramètre estimé est inférieur à au moins un seuil de référence ;
   (d) Estimation de la position de l'objet (1) par fusion des positions déterminées du capteur (2) pour chaque générateur (3) sélectionné dans ledit sous-ensemble.

2. Procédé selon la revendication 1, dans lequel le capteur (2) et les générateurs (3) sont chacun constitué de trois bobines organisées en triaxes.

3. Procédé selon l'une des revendications 1 et 2, dans lequel l'étape (b) comprend pour chaque générateur (3) l'estimation des valeurs de deux invariants mathématiques en fonction des mesures de champ magnétique acquises par le capteur (2) et associées audit générateur (3).

4. Procédé selon la revendication 3, dans lequel ledit paramètre représentatif d'une erreur sur ladite position déterminée du capteur (2) est calculé pour chaque générateur (3) à l'étape (b) en fonction des valeurs estimées desdits deux invariants mathématiques pour ledit générateur (3) et de valeurs théoriques desdits deux invariants mathématiques.

5. Procédé selon l'une des revendications 1 à 4, dans lequel ledit paramètre représentatif d'une erreur sur ladite position déterminée du capteur (2) est une estimation de ladite erreur sur ladite position déterminée du capteur (2).

**6.** Procédé selon l'une des revendications 1 à 5, dans lequel, si ledit sous-ensemble comprend moins qu'un nombre minimal prédéterminé de générateurs (3), ledit sous-ensemble de l'ensemble des générateurs (3) sélectionné à l'étape (c) comprend en outre chaque générateur (3) pour lequel ledit paramètre estimé est inférieur à au moins un seuil dégradé supérieur au seuil de référence

**7.** Procédé selon l'une des revendications 1 à 6, dans lequel la position obtenue par fusion est la position moyenne des positions déterminées du capteur (2) pour chaque générateur (3) sélectionné dans ledit sous-ensemble.

**8.** Procédé selon les revendications 6 et 7 en combinaison, dans lequel, si ledit sous-ensemble de l'ensemble des générateurs (3) sélectionné à l'étape (c) comprend au moins un générateur (3) pour lequel ledit paramètre estimé est supérieur audit seuil de référence, la position obtenue par fusion est la position moyenne des positions déterminées du capteur (2) pour chaque générateur (3) du plus grand sous-ensemble dudit sous-ensemble des générateurs (3) pour lesquels ledit paramètre estimé reste inférieur audit seuil de référence.

**9.** Procédé selon l'une des revendications 1 à 8, dans lequel l'attitude est prise en compte en surplus de la position dans les étapes (a) à (d).

**10.** Système comprenant un capteur magnétique triaxe (2) un ensemble d'au moins trois générateurs magnétiques triaxe (3) statiques dans un référentiel commun, pour la localisation d'un objet (1) solidaire du capteur (2) et évoluant dans le champ magnétique généré par lesdits générateurs (3), le système comprenant des moyens de traitement de données (21) configurés pour mettre en œuvre :

- Un module de détermination, pour chaque générateur (3), d'une position dudit capteur (2) dans ledit référentiel commun en fonction de mesures de champ magnétique acquises par le capteur (2) et associées audit générateur (3) ;

**caractérisé en ce que** le système comprend des moyens de traitement de données (21) configurés pour mettre en oeuvre :

- Un module de calcul, pour chaque générateur (3), d'un paramètre représentatif d'une erreur sur ladite position déterminée du capteur (2) pour le générateur (3) ;
- Un module de sélection d'un sous-ensemble de l'ensemble des générateurs (3) en fonction desdits paramètres estimés pour chacun des générateurs (3) ;
- Un module d'estimation de la position de l'objet (1) par fusion des positions déterminées du capteur (2) pour chaque générateur (3) sélectionné dans ledit sous-ensemble.

**11.** Produit programme d'ordinateur comprenant des instructions de code pour l'exécution d'un procédé de localisation d'un objet (1) solidaire d'un capteur magnétique triaxe (2), évoluant dans un champ magnétique généré par un ensemble d'au moins trois générateurs magnétiques triaxe (3) statiques dans un référentiel commun, selon l'une des revendications 1 à 9, lorsque ledit programme est exécuté sur un ordinateur.

**12.** Moyen de stockage lisible par un équipement informatique sur lequel un produit programme d'ordinateur comprend des instructions de code pour l'exécution d'un procédé de localisation d'un objet (1) solidaire d'un capteur magnétique triaxe (2), évoluant dans un champ magnétique généré par un ensemble d'au moins trois générateurs magnétiques triaxe (3) statiques dans un référentiel commun, selon l'une des revendications 1 à 9.

**Patentansprüche**

**1.** Verfahren zur Ortung eines Objektes (1), das mit einem triaxialen Magnetsensor (2) fest verbunden ist, das sich in einem Magnetfeld bewegt, das von einer Anordnung aus mindestens drei statischen triaxialen Magnetgeneratoren (3) in einem gemeinsamen Referenzrahmen erzeugt wird, wobei das Verfahren den folgenden Schritt umfasst:

(a) Bestimmen, für jeden Generator (3), in Abhängigkeit von Magnetfeldmessungen, die von dem Sensor (2) erfasst und dem Generator (3) zugeordnet werden, einer Position des Sensors (2) im gemeinsamen Referenzrahmen;

wobei das Verfahren **dadurch gekennzeichnet ist, dass** es die folgenden Schritte umfasst:

(b) Berechnen, für jeden Generator (3), eines Parameters, der für einen Fehler auf der bestimmten Position des Sensors (2) für den Generator (3) repräsentativ ist;

(c) Auswählen einer Unteranordnung aus der Anordnung der Generatoren (3) in Abhängigkeit von den für jeden der Generatoren (3) geschätzten Parametern, wobei die aus der Anordnung der Generatoren (3) ausgewählte Unteranordnung jeden Generator (3) umfasst, für den der geschätzte Parameter kleiner als mindestens ein Referenzgrenzwert ist;

(d) Schätzen der Position des Objektes (1) durch Zusammenführen der für jeden aus der Unteranordnung ausgewählten Generator (3) bestimmten Positionen des Sensors (2).

2. Verfahren nach Anspruch 1, wobei der Sensor (2) und die Generatoren (3) jeweils aus drei triaxial organisierten Spulen bestehen.

3. Verfahren nach einem der Ansprüche 1 und 2, wobei der Schritt (b) für jeden Generator (3) die Schätzung der Werte von zwei mathematischen Invarianten in Abhängigkeit der von dem Sensor (2) erfassten und dem Generator (3) zugeordneten Magnetfeldmessungen umfasst.

4. Verfahren nach Anspruch 3, wobei der Parameter, der für einen Fehler auf der bestimmten Position des Sensors (2) repräsentativ ist, für jeden Generator (3) in Schritt (b) in Abhängigkeit von den von den zwei mathematischen Invarianten für den Generator (3) geschätzten Werten und von theoretischen Werten der zwei mathematischen Invarianten berechnet wird.

5. Verfahren nach einem der Ansprüche 1 bis 4, wobei der für einen Fehler auf der bestimmten Position des Sensors (2) repräsentative Parameter eine Schätzung des Fehlers auf der bestimmten Position des Sensors (2) ist.

6. Verfahren nach einem der Ansprüche 1 bis 5, wobei, wenn die Unteranordnung weniger als eine vorbestimmte minimale Anzahl von Generatoren (3) umfasst, umfasst die in Schritt (c) aus der Anordnung der Generatoren (3) ausgewählte Unteranordnung ferner jeden Generator (3), für den der geschätzte Parameter kleiner als mindestens ein verminderter Schwellenwert ist, der größer als der Referenzgrenzwert ist.

7. Verfahren nach einem der Ansprüche 1 bis 6, wobei die durch Zusammenführen erhaltene Position die mittlere Position der für jeden ausgewählten Generator (3) in der Unteranordnung bestimmten Positionen des Sensors (2) ist.

8. Verfahren nach den Ansprüchen 6 und 7 in Kombination, wobei, wenn die in Schritt (c) aus der Anordnung der Generatoren (3) ausgewählte Unteranordnung mindestens einen Generator (3) umfasst, für den der geschätzte Parameter größer als der Referenzgrenzwert ist, ist die durch Zusammenführen erhaltene Position die mittlere Position der für jeden Generator (3) der größten Unteranordnung aus der Unteranordnung der Generatoren (3) bestimmten Positionen des Sensors (2), für die der geschätzte Parameter kleiner als der Referenzgrenzwert bleibt.

9. Verfahren nach einem der Ansprüche 1 bis 8, wobei in den Schritten (a) bis (d) neben der Position die Haltung berücksichtigt wird.

10. System, umfassend einen triaxialen Magnetsensor (2) und eine Anordnung aus mindestens drei statischen triaxialen Magnetgeneratoren (3) in einem gemeinsamen Referenzrahmen für die Ortung eines Objektes (1), das mit dem Sensor (2) fest verbunden ist und sich in dem von den Generatoren (3) erzeugten Magnetfeld bewegt, wobei das System Datenverarbeitungsmittel (21) umfasst, die ausgelegt sind, um umzusetzen:

- ein Modul zum Bestimmen, für jeden Generator (3), in Abhängigkeit von Magnetfeldmessungen, die von dem Sensor (2) erfasst und dem Generator (3) zugeordnet werden, einer Position des Sensors (2) im gemeinsamen Referenzrahmen;

**dadurch gekennzeichnet, dass** das System Datenverarbeitungsmittel (21) umfasst, die ausgelegt sind, um umzusetzen:

- ein Modul zum Berechnen, für jeden Generator (3), eines Parameters, der für einen Fehler auf der bestimmten Position des Sensors (2) für den Generator (3) repräsentativ ist;
- ein Modul zum Auswählen einer Unteranordnung aus der Anordnung der Generatoren (3) in Abhängigkeit von den für jeden der Generatoren (3) geschätzten Parametern;
- ein Modul zum Schätzen der Position des Objektes (1) durch Zusammenführen der für jeden aus der Unteranordnung ausgewählten Generator (3) bestimmten Positionen des Sensors (2).

11. Rechnerprogrammprodukt, umfassend Codebefehle für die Ausführung eines Verfahrens für die Ortung eines Objektes (1), das mit einem triaxialen Magnetsensor (2) fest verbunden ist, das sich in einem von einer Anordnung von mindestens drei statischen triaxialen Magnetgeneratoren (3) in einem gemeinsamen Referenzrahmen erzeugten Magnetfeld bewegt, nach einem der Ansprüche 1 bis 9, wenn das Programm auf einem Rechner ausgeführt wird.

12. Speichermittel, das von einer IT-Ausrüstung lesbar ist, auf der ein Rechnerprogrammprodukt Codebefehle für die Ausführung eines Verfahrens für die Ortung eines Objektes (1) umfasst, das mit einem triaxialen Magnetsensor (2) fest verbunden ist, das sich in einem von einer Anordnung von mindestens drei statischen triaxialen Magnetgeneratoren (3) in einem gemeinsamen Referenzrahmen erzeugten Magnetfeld bewegt, nach einem der Ansprüche 1 bis 9.

**Claims**

1. Method for locating an object (1) integral with a triaxial magnetic sensor (2), moving in a magnetic field generated by a set of at least three triaxial magnetic generators (3) static in a common reference frame, the method comprising the step of :

   (a) For each generator (3), determination of a position of the said sensor (2) in the said common reference frame as a function of magnetic field measurements acquired by the sensor (2) and associated with the said generator (3);

   the process being **characterized in that** it comprises the steps of:

   (b) Calculation for each generator (3) of a parameter representative of an error on said determined position of the sensor (2) for the generator (3);
   (c) Selection of a subset of the set of generators (3) as a function of said parameters estimated for each of the generators (3), said selected subset of the set of generators (3) comprising each generator (3) for which said estimated parameter is less than at least one reference threshold;
   (d) Estimation of the position of the object (1) by merging the determined positions of the sensor (2) for each generator (3) selected in said subset.

2. Method according to claim 1, wherein the sensor (2) and the generators (3) each consist of three coils arranged in triaxes.

3. Method according to one of claims 1 and 2, in which step (b) comprises for each generator (3) the estimation of the values of two mathematical invariants as a function of the magnetic field measurements acquired by the sensor (2) and associated with said generator (3).

4. Method according to claim 3, wherein said parameter representative of an error on said determined position of the sensor (2) is calculated for each generator (3) in step (b) as a function of the estimated values of said two mathematical invariants for said generator (3) and theoretical values of said two mathematical invariants.

5. Method according to any one of claims 1 to 4, wherein said parameter representative of an error on said determined position of the sensor (2) is an estimation of said error on said determined position of the sensor (2).

6. Method according to any one of claims 1 to 5, wherein, if said subset comprises less than a predetermined minimum number of generators (3), said subset of the set of generators (3) selected in step (c) further comprises each generator (3) for which said estimated parameter is less than at least one degraded threshold greater than the reference threshold.

7. Method according to any one of claims 1 to 6, wherein the position obtained by merging is the average position of the determined positions of the sensor (2) for each generator (3) selected in said subset.

8. Method according to claims 6 and 7 in combination, wherein, if said subset of the set of generators (3) selected in step (c) comprises at least one generator (3) for which said estimated parameter is greater than said reference threshold, the position obtained by merging is the average position of the determined positions of the sensor (2) for each generator (3) of the largest subset of said subset of generators (3) for which said estimated parameter remains less than said reference threshold.

9. Method according to any one of claims 1 to 8, wherein the attitude is taken into account in addition to the position in steps (a) to (d).

10. System comprising a triaxial magnetic sensor (2), a set of at least three triaxial magnetic generators (3) static in a common reference frame, for locating an object (1) integral with the sensor (2) and moving in the magnetic field generated by said generators (3), the system comprising data processing means (21) configured to implement:

 - a module for determining, for each generator (3), a position of said sensor (2) in said common reference frame as a function of magnetic field measurements acquired by the sensor (2) and associated with said generator (3);

 **characterised in that** the system comprises data processing means (21) configured to implement:

 - a module for calculating, for each generator (3), a parameter representative of an error on said determined position of the sensor (2) for the generator (3);
 - a module for selecting a subset of the set of generators (3) as a function of said estimated parameters for each of the generators (3);
 - a module for estimating the position of the object (1) by merging the determined positions of the sensor (2) for each generator (3) selected in said subset.

11. Computer program product comprising code instructions for executing a method for locating an object (1) integral with a triaxial magnetic sensor (2), moving in a magnetic field generated by a set of at least three triaxial magnetic generators (3) static in a common reference frame, according to one of claims 1 to 9, when said program is executed on a computer.

12. Storage means readable by a computer equipment on which a computer program product comprises code instructions for executing a method for locating an object (1) integral with a triaxial magnetic sensor (2), moving in a magnetic field generated by a set of at least three triaxial magnetic generators (3) static in a common reference frame, according to one of claims 1 to 9.

FIG. 1

FIGURE 2

FIG. 2

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3868565 A
- US 4737794 A
- US 4945305 A
- US 6636757 B
- US 6400139 B
- US 2011224537 A
- US 2011004430 A
- US 5307072 A